# EUROPEAN PATENT SPECIFICATION

(11) **EP 1 956 986 B1**
(45) Date of publication and mention of the grant of the patent: **29.03.2017**
(21) Application number: 06838710.9
(22) Date of filing: 01.12.2006
(51) Int. Cl.: A61B 17/00

(54) **DEVICES, SYSTEMS, AND METHODS FOR OCCLUDING A DEFECT**
VORRICHTUNGEN, SYSTEME UND VERFAHREN ZUM VERSCHLUSS EINES DEFEKTS
DISPOSITIFS, SYSTEMES ET PROCEDES POUR BOUCHER UN DEFAUT

(30) Priority: 02.12.2005 US 741823 P
(43) Date of publication of application: 20.08.2008
(73) Proprietor: Cook Medical Technologies LLC, Bloomington, IN 47404 (US)
(72) Inventor: ARMSTRONG, David, Atlanta, GA 30350 (US)
(74) Representative: Garratt, Peter Douglas
(86) International application number: PCT/US2006/045890
(87) International publication number: WO 2007/064819

(56) References cited:
- WO-A-2006/119256
- WO-A2-02/062234
- US-A- 5 522 840
- US-A1- 2005 159 776

## Description

### RELATED APPLICATIONS

### FIELD OF THE INVENTION

The present invention relates generally to medical devices, systems, and methods, and in particular aspects to medical devices, systems, and methods for treating defects.

### BACKGROUND OF THE INVENTION

A variety of defects, or abnormal passages, can occur in a mammalian body. Such defects may be caused by, for example, an infection, a congenital defect, inflammatory bowel disease (such as Crohn's disease), irradiation, trauma, neoplasia, childbirth, or a side effect from a surgical procedure.

Some defects occur as fistulas between the vagina and the bladder (vesico-vaginal fistulas) or between the vagina and the urethra (urethro-vaginal fistulas). These fistulas may be caused by trauma during childbirth. Traditional surgery for these types of fistulas is complex and not very successful.

Other fistulas include, but are not limited to, tracheo-esophageal fistulas, gastro-cutaneous fistulas, fistulas extending between the vascular and gastrointestinal systems, between the small bowel and skin (entero-cutaneous fistulas), between the stomach and skin (gastro-cutaneous fistulas), between the colon and skin (colo-cutaneous fistulas), between the vagina and bladder (vesico-vaginal fistulas), between the vagina and urethra (urethra-vaginal fistulas), any number of anorectal (ano-cutaneous) fistulas, such as fistulas that form between the anorectum and vagina (recto-vaginal fistulas), between the anorectum and bladder (recto-vesical fistulas), between the anorectum and urethra (recto-urethral fistulas), or between the anorectum and prostate (recto-prostatic fistulas), or fistulas extending between any other two portions of the body. Anorectal fistulas, for example, can result from infection in the anal glands, which are located around the circumference of the distal anal canal forming an anatomic landmark known as the dentate line. Approximately 20-30 such glands are found in humans. Infection in an anal gland can result in an abscess. This abscess can then track through soft tissues (e.g., through or around the sphincter muscles) and into the perianal skin, where it drains either spontaneously or surgically. The resulting void through the soft tissue is known as a fistula. Fistulas may take various paths, which vary in complexity. The internal or inner opening of the fistula, usually located at or near the dentate line, is known as the primary opening. The primary opening is usually the high pressure end of a fistula. Any external or outer openings, which are usually located in the perianal skin, are known as the secondary openings. The secondary openings are usually the low pressure end of a fistula.

Other types of defects include, but are not limited to, defects in solid organs, such as bleeding biopsy sites in the liver or perineal sinuses typically observed in patients with Crohn's disease; defects in vessels, such as arteries or veins in which hemorrhage needs to be arrested or blood flow needs to be diverted from one area to another; and viscera containing persistent air leaks from a bronchial stump after surgical pneumonectomy or lobectomy.

Typical techniques for treating a defect such as a fistula involve draining infection from the fistula tract and maturing it prior to a definitive closure or sealing procedure by inserting a narrow diameter rubber drain, known as a seton, through the tract. This is usually accomplished by inserting a fistula probe through the outer (secondary) opening and gently guiding it through the fistula, and out through the inner (primary) opening. A seton, thread or tie is then affixed to the tip of the probe, which is then withdrawn from the tract, leaving the seton in place. The seton may then be tied as a loop around the contained tissue and left for several weeks or months.

One technique for treating a defect is to occlude the defect with an occluding member, such as a plug or graft. Examples of such occluding members and related methods are disclosed in co-pending U.S. Application Serial Nos. 10/945,634 (Armstrong), 11/040,996 (Armstrong), and 11/472,642 (Armstrong et al.), and U.S. Provisional Application Serial Nos. 60/763,550; 60/763,521; 60/676,482; 60/676,118; and 60/839,976. For example, an occluding member may be pulled through the primary opening of a fistula until the occluding member is securely lodged within the fistula. The occluding member may be further secured within the fistula by the use of sutures or a capping member associated with the body of the occluding member.

Another technique for treating a fistula involves the use of a plug-like closure device in combination with a drainage thread or seton, as disclosed in co-pending U.S. Application Serial No. 10/911,958 (Burgard). In this technique, a closure device is provided with a flexible application string that can be used to drain secretions or other undesirable liquids from the fistula. A rod-like instrument is pushed into the fistula from the outer opening and is used to investigate the trajectory of the fistula. After the instrument is pushed forward enough to protrude from the inner opening, the application string is pulled through the fistula from the inner opening until the closure device "sticks" in the inner opening. The closure device is then pushed as far as necessary for it to be tightly secured within the fistula.

Still other techniques for treating fistulas are described in U.S. Application Serial No. 11/415,403, titled "VOLUMETRIC GRAFTS FOR TREATMENT OF FISTULAE AND RELATED METHODS AND SYSTEMS" (Cook Biotech Incorporated), filed May 1,2006; and U.S. Provisional Application Serial No. 60/815,502, titled "FISTULA GRAFTS AND RELATED METHODS AND SYSTEMS USEFUL FOR TREATING GASTROINTESTINAL FISTULAE" (Cook Biotech Incorporated, assignee), filed June 21, 2006.

The above techniques can be difficult for some physicians to perform, especially when the defect to be treated is located in an area that is not easily accessible. Therefore, there remains a need for simplified procedures and new medical devices and systems for treating defects. The present invention addresses these needs.

Reference is directed to US 5522840 which discloses a device for the non-surgical seal of the interstices in the wall of a vessel comprising the features defined in the preamble of independent claim 1.

### SUMMARY OF THE INVENTION

The present invention provides devices for occluding fistulas that overcome several shortcomings of the prior art and simplify the implantation of an occluding member in a defect of a patient.

The scope of the present invention is set forth in the appended claims. The present invention may be used to occlude any type of defect, or abnormal bodily passage. For example, the claimed devices may be used to occlude trached-esophageal fistulas, gastro-cutaneous fistulas, anorectal fistulas, fistulas occurring between the vagina and the urethra or bladder, fistulas occurring between the anorectum and vagina, fistulas occurring between the vascular and gastrointestinal systems, defects in solid organs, defects in vessels, viscera containing persistent air leaks, or any other type of defect.

In one aspect, a medical device for occluding a defect is disclosed. The medical device may comprise an occluding member, such as a plug or graft, configured to be placed within a fistula and to occlude the fistula. The medical device may further comprise a lumen configured to receive a wire guide. In some embodiments, the lumen extends throughout the length of the occluding member and is positioned in or near the center of the occluding member. A longitudinal slit may be positioned adjacent to the lumen to permit lateral insertion of the wire guide. The device may be made of any biocompatible material and may be of any suitable shape, dimension, and material for at least partially occluding a defect. In some desirable embodiments, the device has a generally conical shape and is made of a remodelable extracellular matrix material, such as small intestinal submucosa.

In another aspect, a system for occluding a fistula is disclosed. In some embodiments, the system comprises a placement member (such as a wire guide), an occluding member (such as a plug or graft) having a first lumen, and a pusher member having a second lumen. Desirably, the occluding member and the pusher member are configured to be received on the wire guide to facilitate insertion of the occluding member into a defect (such as a fistula). The occluding member may be of any suitable shape, dimension, and material for at least partially occluding a defect, and the wire guide and pusher member may be of any suitable shape, dimension, and material for facilitating the delivery of the occluding member to its desired location.

In still another aspect, a method of occluding a defect is disclosed. In some embodiments, the method comprises the steps of: (a) providing a placement member (such as a wire guide), an occluding member (such as a plug or graft) having a first lumen, and a pusher member having a second lumen; (b) inserting the placement member at least partially into the defect; (c) advancing the placement member to a desired location; (d) inserting the placement member into the first lumen of the occluding member; and (e) advancing the occluding member to the desired location by inserting the placement member into the second lumen of the pusher member and pushing the occluding member with the pusher member until the occluding member reaches the desired location. In some embodiments, the placement member is an endoscope or a hollow tubular device that may be used to inject contrast solution into the defect. The placement member may be inserted into the occluding member by threading it through a lumen in the occluding member or by moving it in a lateral direction through a slit in the external surface of the occluding member and then moving it into the lumen. The method may also include anchoring the occluding member within the defect by any suitable means, injecting a rehydrating fluid into the tissues surrounding the defect to rehydrate a dehydrated occluding member, and/or trimming any excess portions of the occluding member to prevent protrusion from the defect. In some embodiments, an endoscope is utilized to assist with visualization and insertion of the placement member into the defect. An instrument channel within the endoscope may be used to facilitate the delivery of wire guides, catheters, medical devices, and the like into the defect during the implantation procedure.

Additional features and advantages of the present invention will be apparent to one of ordinary skill in the art from the drawings and detailed description of the preferred embodiments below. Moreover, it should be appreciated that several aspects of the present invention can be performed with alternative types of wire guides, catheters, endoscopes, occluding members, pusher members, and other medical devices.

### BRIEF DESCRIPTION OF THE DRAWINGS

Figure 1 is a perspective view of a medical device, where the medical device comprises an occluding member having a curved, generally conical body, and where a wire guide has been inserted through a lumen in the occluding member;
Figure 2 is a perspective view of another medical device, where the medical device comprises an occluding member having a generally conical body and a central lumen extending throughout the length of the occluding member, and where a wire guide has been inserted through a lumen in the occluding member;
Figure 3 is a perspective view of still another medical device similar to Figure 2, but including a longitudinal slit located adjacent to the lumen to facilitate lateral insertion of a placement member, such as a wire guide, into the lumen;
Figure 4 is a side view of yet another medical device, where the medical device comprises an occluding member having a generally cylindrical body, a lumen extending throughout the length of the occluding member; and a capping member to assist with anchoring the device within a defect;
Figure 5 is a perspective view of the device of Figure 2 in which a pusher member has been threaded over the wire guide and is being advanced distally along the wire guide;
Figure 6 shows a medical device implanted within an anorectal fistula.

### DETAILED DESCRIPTION OF THE PREFERRED EMBODIMENT

While the present invention may be embodied in many different forms, for the purpose of promoting an understanding of the principles of the present invention, reference will now be made to the embodiments illustrated in the drawings, and specific language will be used to describe the same. It will nevertheless be understood that no limitation of the scope of the invention is thereby intended. Any alterations and further modifications in the described embodiments and any further applications of the principles of the present invention as described herein are contemplated as would normally occur to one skilled in the art to which the invention relates.

Turning now to a discussion of particular embodiments of the medical devices, systems, and methods of the present invention useful for treating defects, illustrative medical devices of the invention are configured to at least partially occlude a defect within a patient. For example, in some embodiments of the invention, a medical device is used to occlude at least the primary opening of a fistula and potentially one or more other segments of the fistula, the fistula tract, and/or any secondary openings. In this context, the term "fistula tract" is meant to include, but is not limited to, a void in the soft tissues extending from a primary fistula opening, whether blind-ending or leading to one or more secondary fistula openings.

The medical devices, systems, and methods of the present invention may be used to occlude any type of defect. For example, defects such as anorectal fistulas, tracheo-esophageal fistulas, gastro-cutaneous fistulas, or fistulas occurring between the vagina and bladder (vesico-vaginal fistulas), between the vagina and urethra (urethro-vaginal fistulas), between the anorectum and vagina (recto-vaginal fistulas), between the anorectum and bladder (recto-vesical fistulas), between the anorectum and urethra (recto-urethral fistulas), between the anorectum and prostate (recto-prostatic fistulas), or between the vascular and gastrointestinal systems, defects in solid organs (such as bleeding biopsy sites in the liver or perineal sinuses typically observed in patients with Crohn's disease), defects in vessels (such as arteries or veins) in which hemorrhage needs to be arrested or blood flow needs to be diverted from one area to another, and viscera containing persistent air leaks from a bronchial stump after surgical pneumonectomy or lobectomy may be treated with the devices, systems, and methods of the present invention.

Generally, the medical devices of the present invention comprise an occluding member configured for implantation into a defect such as a fistula. The occluding member may have any suitable shape, configuration, and dimensions, such as those disclosed in co-pending U.S. Application Serial Nos. 10/911,958 (Burgard), 10/945,634 (Armstrong), 11/040,996 (Armstrong), 11/415,403 (Cook Biotech Incorporated, assignee), and 11/472,642 (Armstrong et al.), U.S. Provisional Application Serial No. 60/763,521 (Cook Biotech Incorporated, assignee), and U.S. Provisional Application Serial No. 60/815,502, titled "FISTULA GRAFTS AND RELATED METHODS AND SYSTEMS USEFUL FOR TREATING GASTROINTESTINAL FISTULAE" (Cook Biotech Incorporated, assignee), filed June 21, 2006. For example, the occluding member may be of any suitable dimensions and may have a body that is generally convex, bi-convex, concave, bi-concave, S-shaped, straight, curved, flat, polygonal, ovoid, conical, cylindrical, elliptical, helical, spherical, or hemispherical, or it may have any other configuration capable of being inserted into and secured within a defect. In certain embodiments, the occluding member comprises a plug or graft that is sufficiently rigid to facilitate delivery into an implantation site, and that has one or more lumens extending at least partially through the plug or graft body along its length. In some embodiments, the occluding member comprises a body having a central lumen to facilitate deployment of the occluding member body over a wire guide or other delivery device. In some embodiments, the body of the occluding member has portions that are tapered and/or curvilinear. In other embodiments, the body of the occluding member is curved to conform to the shape of the fistula, thereby facilitating introduction of the occluding member, a secure fit of the occluding member within the fistula, and less discomfort for the patient.

The body of the occluding member of the present invention may have any dimension suitable for implantation within a defect of a patient. In some embodiments, the body of the occluding member has a size and shape adapted to extend into at least a portion of a fistula tract, and is generally (but not necessarily) of sufficient dimension to fill a fistula, or a segment thereof, e.g., the primary fistula opening, fistula tract, and/or any secondary fistula openings, either alone or in combination with other components of the occluding member and/or other similar or differing medical devices. The body of the occluding member may or may not be sized and shaped to fill the entire defect.

The occluding member includes a lumen. The lumen may have any shape or configuration suitable for receiving a placement member, such as a wire guide, and may be positioned in any suitable location within the medical device. Desirably, the lumen is a cylindrical channel that extends throughout the length of the occluding member, and is disposed in or near the center of the occluding member body. In certain embodiments, the occluding member also contains a longitudinal slit extending from the exterior surface of the occluding member to the lumen. Such a slit may facilitate lateral insertion of the placement member into the lumen of the occluding member. The lumen and the longitudinal slit may have any suitable dimensions appropriate for use with a placement member. For example, in desirable embodiments, the lumen disposed in the occluding member body has an inner diameter of about 0.1 mm to about 5 mm. More desirably, the lumen has an inner diameter of about 0.2 mm to about 2 mm, and even more desirably, the lumen has an inner diameter of about 0.5 mm to about 1 mm.

In addition to an occluding member body, the medical devices of the present invention may include other components that are integrally incorporated into the medical device as single unitary construct or configured as separate components that are associated with the occluding member body in any suitable manner. For example, a capping member may be integral with, attached to, or otherwise associated with the body of the occluding member, as described in co-pending U.S. Application Serial No. 11/472,642 (Armstrong et al.). The capping member may be used to prevent unintentional displacement of the occluding member after implantation. In some embodiments, the capping member is configured to contact portions of an alimentary canal wall adjacent to the primary opening of an anorectal fistula, and the body of the occluding member is configured to extend into at least a portion of the fistula tract. In other embodiments, a second cap (which may be expandable) configured to contact portions of the tissue adjacent to a secondary opening is associated with or attached to the body of the occluding member before, during, or after implantation. In still other embodiments, the medical device of the present invention also includes an elongated tail, which may be used to facilitate deployment of the occluding member and to eliminate the need for a separate seton placement step in the implantation procedure.

In certain embodiments of the present invention, the medical device includes a coupling structure. The coupling structure may have any suitable configuration and dimension for implantation into a defect of a patient. In some embodiments, the coupling structure is configured to engage a delivery device (e.g., a pusher member or placement member, such as a wire guide). Desirably, the coupling structure is configured to be easily attached to a delivery device and to remain attached to the delivery device while force is exerted on the delivery device and attached medical device to properly position the medical device within a patient. The coupling structure may also be configured for easy detachment from the delivery device after the medical device is properly positioned within the patient.

In certain embodiments of the present invention, the medical device includes an anchoring adaptation to prevent displacement of the medical device and/or its components following implantation of the medical device. For example, the medical device may have protrusions on its outer surface to assist in anchoring the medical device within a defect, or it may have other suitable anchoring adaptations, including but not limited to barbs, hooks, sutures, adhesives, ribs, and the like. Such anchoring adaptations, while advantageous in certain embodiments of the invention, are not necessary to broader aspects of the invention. Illustratively, certain medical devices are configured so that a capping member is used to maintain contact with the tissue adjacent to the primary opening of a fistula following implantation, thereby eliminating the need for other anchoring adaptations, as disclosed in U.S. Application Serial No. 11/472,642 (Armstrong et al.). In other embodiments of the invention, suitable anchoring adaptations may aid or facilitate the maintenance of such contact.

In some aspects of the invention, a system for occluding a defect is provided. In certain embodiments, the system comprises an occluding member (e.g., a plug or graft) having a lumen, a pusher member having a lumen, and a placement member *(e.g.,* a wire guide or similar device). In some embodiments, the lumen of the occluding member is located in or near the center of the occluding member. The occluding member may also include a longitudinal slit in its external surface, adjacent to the lumen, to facilitate lateral insertion of the placement member into the lumen of the occluding member. Desirably, the occluding member and the pusher member are configured to be received on the placement member to facilitate insertion of the plug into the defect.

The placement member may have any suitable shape, configuration, and dimensions for facilitating delivery of a medical device into a defect of a patient. In some embodiments of the system of the present invention, the placement member is a wire guide or endoscope. The placement member may be rigid, semi-rigid, flexible, or any combination thereof. In certain embodiments, the placement member comprises a hollow tubular device that is configured to inject a contrast solution into the defect to facilitate visualization and implantation of the medical device.

The pusher member may have any suitable shape, configuration, and dimensions for advancing a medical device along a placement member and into a defect. In desirable embodiments, the pusher member is sufficiently flexible to navigate any curvature present in the defect of a patient. The dimensions of the pusher member may vary with the dimensions of the occluding member and the placement member. For example, in some embodiments, a wire guide having a diameter of about 0.1 mm to about 1 mm and a length of about 50 cm to about 300 cm is used with a pusher member having an inner diameter of about 0.25 mm to about 1.5 mm and a length of about 10 cm to about 150 cm. In other embodiments, a wire guide having a diameter of about 0.5 mm and a length of about 100 cm is used with a pusher member having an inner diameter of about 0.75 mm to about 1 mm and a length of about 25 cm to about 75 cm.

The body of the occluding member and/or any other components of the medical device or system of the present invention may be made of any biocompatible material suitable for implantation into a mammalian body. Desirably, the biocompatible material comprises a biocompatible biological material (e.g., a heterograft, allograft, or autograft material) or a biocompatible synthetic material. More desirably, the material comprises a tissue ingrowth material, which facilitates incorporation of the host tissue of the patient into the body of the occluding member and/or other components of the medical device after implantation. A detailed description of and listing of non-limiting illustrative examples of suitable materials for use in the present invention are provided in co-pending U.S. Application Serial No. 11/472,642 (Armstrong et al.). In some embodiments, a sheet form material that is deformable upon impingement by soft tissue is used to form one or more of the components of the medical device. In some embodiments, the material has a collagenous tissue frame that remains intact to allow for ingrowth of host cells and eventual reconstruction of the host tissue itself. Desirable remodelable collagenous materials can be provided, for example, by collagenous materials isolated from a warm-blooded vertebrate, and especially a mammal. Such isolated collagenous material can be processed so as to have remodelable, angiogenic properties and promote cellular invasion and ingrowth. Remodelable materials may be used in this context to promote cellular growth on, around, and/or within tissue in which a medical device of the invention is implanted, e.g., around tissue defining a fistula tract or an opening to a fistula.

Suitable remodelable materials include, but are not limited to, collagenous extracellular matrix (ECM) materials, which are described more fully in co-pending U.S. Application Serial No. 11/472,642 (Armstrong et al.). In some embodiments of the present invention, naturally-derived ECM materials are used. In other embodiments, synthetic remodelable/regenerative ECM materials are used. ECM material used in the present invention may be free of additional non-native crosslinking, or may contain additional crosslinking. Examples of suitable collagenous materials include, but are not limited to, ECM materials such as submucosa, renal capsule membrane, dermal collagen, dura mater, pericardium, serosa, and peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. Submucosa useful in the present invention can be obtained by harvesting such tissue sources and delaminating the submucosa from smooth muscle layers, mucosal layers, and/or other layers occurring in the tissue source. For additional information as to submucosa useful in the present invention, and its isolation and treatment, reference can be made, for example, to U.S. Patent Nos. 4,902,508, 5,554,389, 5,993,844, 6,206,931, 6,099,567, and 6,331,319, and PCT Publication WO03002165.

When formed separately, the components of the medical devices and systems of the present invention may or may not be comprised of the same biocompatible material(s) as the other components of the device or system. In certain aspects, the components are formed from separate pieces of material, yet are retained in association with one another without the use of any other device or material (e.g., sutures, an adhesive, etc.). For example, the body of the occluding member and the tail (if present) may be held together by having at least one member (or any portion thereof) received around, through, over, etc., the other member or any portion thereof. In some embodiments, a single component of the medical device of the present invention may comprise one or more types of material. For example, an occluding member body may be made of a multilaminate material comprising a plurality of layers of a single material or of multiple, different materials, where the layers may be bonded together in any suitable manner (e.g., by a bonding agent, cross-linking, or vacuum pressing).

In some embodiments of the present invention, one or more bioactive agents are included. As used herein, the phrase "bioactive agent" refers to any pharmaceutically active agent that produces an intended therapeutic effect on the body to treat or prevent conditions or diseases. Such bioactive agents may be incorporated into the medical device, coated onto the medical device, or included in the medical device (or portions thereof) in any other suitable manner. For example, a bioactive agent (or a bioactive agent combined with another biocompatible material) may be coated onto the body of the medical device and configured to release over a certain period of time.

Suitable bioactive agents may include one or more bioactive agents native to the source of an ECM tissue material. For example, a submucosa or other remodelable ECM tissue material may retain one or more growth factors including but not limited to basic fibroblast growth factor (FGF-2), transforming growth factor beta (TGF-beta), epidermal growth factor (EGF), cartilage derived growth factor (CDGF), and/or platelet derived growth factor (PDGF). In addition, submucosa or other ECM materials when used in the invention may retain other native bioactive agents including but not limited to proteins, glycoproteins, proteoglycans, and glycosaminoglycans. For example, ECM materials may include heparin, heparin sulfate, hyaluronic acid, fibronectin, cytokines, and the like. Thus, generally speaking, a submucosa or other ECM material may retain one or more bioactive components that induce, directly or indirectly, a cellular response such as a change in cell morphology, proliferation, growth, protein or gene expression.

In addition or as an alternative to the inclusion of such native bioactive components, non-native bioactive components such as those synthetically produced by recombinant technology or other methods (e.g., genetic material such as DNA), may be incorporated into the material used to form the components of the medical device of the present invention. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to material layers include, for example, anti-clotting agents, e.g. heparin, antibiotics, anti-inflammatory agents, and anti-proliferative agents, e.g. taxol derivatives such as paclitaxel. Such non-native bioactive components can be incorporated into and/or onto a material in any suitable manner, such as by surface treatment (*e.g.,* spraying) and/or impregnation (*e.g.,* soaking), just to name a few non-limiting examples.

Other suitable bioactive agents that may be used in the present invention include, but are not limited to: antithrombotics, antiplatelets, fibrinolytics, antiproliferative/antimitotic agents, antiplatelet agents, antiproliferative/antimitotic alkylating agents, antiproliferative/antimitotic antimetabolites, platinum coordination complexes, hormones, anticoagulants, fibrinolytic agents, antimigratory agents; antisecretory agents; anti-inflammatory agents, para-aminophenol derivatives, indole and indene acetic acids, immunosuppressives, angiogenic agents, angiotensin receptor blockers, nitric oxide and nitric oxide donors, anti-sense oligionucleotides and combinations thereof, cell cycle inhibitors, retenoids, cyclin/CDK inhibitors, endothelial progenitor cells (EPC), angiopeptin, pimecrolimus, angiopeptin, HMG co-enzyme reductase inhibitors, metalloproteinase inhibitors, protease inhibitors, antibodies, and Liposomal Biphosphate Compounds (BPs). Additional illustrative examples of suitable bioactive agents that may be used in the present invention are set forth in U.S. Application Serial No. 11/472,642 (Armstrong et al.).

Medical devices of the present invention may also comprise a variety of synthetic polymeric materials including but not limited to bioresorbable and/or non-bioresorbable plastics. Bioresorbable, or bioabsorbable polymers that may be used include, but are not limited to, poly(L-lactic acid), polycaprolactone, poly(lactide-co-glycolide), poly(hydroxybutyrate), poly(hydroxybutyrate-co-valerate), polyethylene terephthalate, polygalactin, hyaluronic acid, polydioxanone, polyorthoester, polyanhydride, poly(glycolic acid), poly(D,L-lactic acid), poly(glycolic acid-co-trimethylene carbonate), polyhydroxyalkanaates, polyphosphoester, polyphosphoester urethane, poly(amino acids), cyanoacrylates, poly(trimethylene carbonate), poly(iminocarbonate), copoly(ether-esters) (e.g., PEO/PLA), polyalkylene oxalates, and polyphosphazenes. These or other bioresorbable materials may be used, for example, where only a temporary blocking or closure function is desired, and/or in combination with non-bioresorbable materials where only a temporary participation by the bioresorbable material is desired.

Non-bioresorbable, or biostable polymers that may be used include, but are not limited to, polytetrafluoroethylene (PTFE) (including expanded PTFE), polyethylene terephthalate (PET), polyurethanes, silicones, and polyesters and other polymers such as, but not limited to, polyolefins, polyisobutylene and ethylene-alphaolefin copolymers; acrylic polymers and copolymers, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones, polyvinyl aromatics, such as polystyrene; polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins; polycarbonates; polyoxymethylenes; polyimides; polyethers; epoxy resins; polyurethanes; rayon; and rayon-triacetate.

Desirably, the biological or synthetic materials used in the present invention assist in reconstruction of the host tissues, elicit little immunological reaction, and have some inherent resistance to infection. Such materials may desirably allow incorporation of the medical device into the host tissue of the fistula (rather than complete absorption of the medical device into the surrounding tissue), thereby occluding the defect in the patient.

The components of a medical device of the present invention (*e*.*g*., occluding member body, tail, capping member(s), anchoring adaptations, and/or coupling structure), whether formed separately or together as a single unit, can be constructed in any suitable manner. In some embodiments, the occluding member body, tail, capping member(s), anchoring adaptations and/or coupling structure are formed with a reconstituted or otherwise reassembled ECM material. Any or all of the components of the medical device may be formed by folding or rolling, or otherwise overlaying one or more portions of a biocompatible material, such as a biocompatible sheet material. The overlaid biocompatible sheet material can be compressed and dried or otherwise bonded into a volumetric shape such that a substantially unitary construct is formed. In some embodiments, a medical device is constructed by randomly or regularly packing one or more pieces of single or multilayer ECM sheet material within a mold and thereafter processing the packed material. Occluding member bodies useful in the present invention can be prepared, for example, as described in U.S. Application Serial No. 11/415,403 (Cook Biotech Incorporated, assignee).

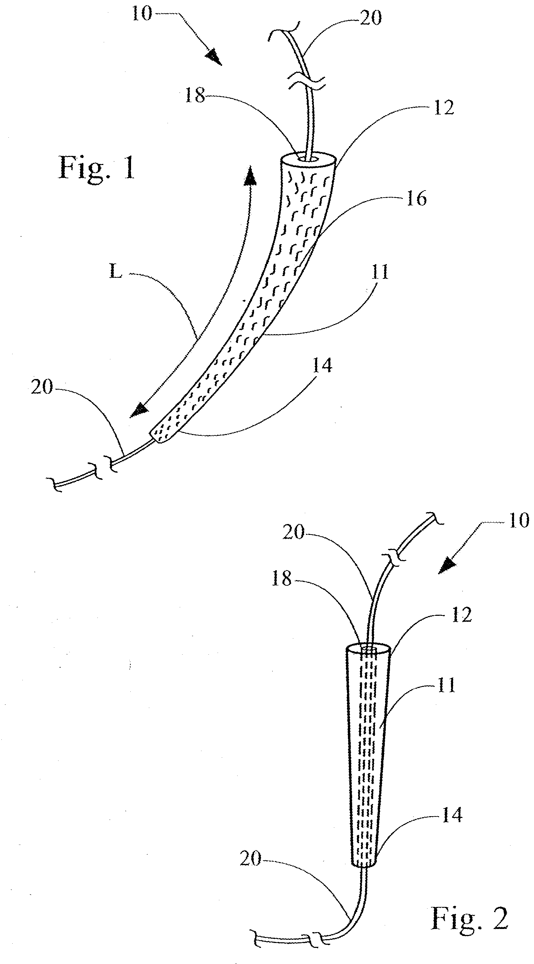

With reference now to the Figures, Figure 1 shows one embodiment of a medical device 10 of the present invention. In this embodiment, the medical device 10 has a slightly curved, generally conical occluding member body 11 having a length L, a proximal end 12, and a distal end 14. As shown, the medical device also includes a lumen 18 positioned in or near the center of the device 10 and anchoring members 16 located on the external surface of the device 10. The anchoring members 16 illustrated in Figure 1 are barbs, but any suitable anchoring mechanism may be used, including but not limited to, capping members, hooks, sutures, adhesives, or ribs. In some embodiments, no anchoring member is necessary to adequately secure the medical device within a defect. In other embodiments, multiple different types of anchoring members may be used. The lumen 18 in the medical device 10 of Figure 1 extends throughout the length L of the medical device 10, from the proximal end 12 to the distal end 14, and is adapted to receive a wire guide 20 therethrough. Although the medical device 10 may be made of any biocompatible material, desirable embodiments comprise an extracellular matrix material, such as small intestinal submucosa.

With reference now to Figure 2, an alternative embodiment of a medical device 10 of the present invention is shown. In this embodiment, the medical device 10 has a generally conical occluding member body 11 and a central lumen 18 extending from the proximal end 12 of the occluding member body 11 to the distal end 14 of the occluding member body. A wire guide 20 may be inserted into the lumen 18 of the occluding member body 11, for example, by placing the distal end 14 of the occluding member body 11 over the proximal end of the wire guide 20 and threading the occluding member body 11 over the wire guide 20 until the wire guide 20 protrudes from the proximal end 12 of the occluding member body 11, as shown in Figure 2.

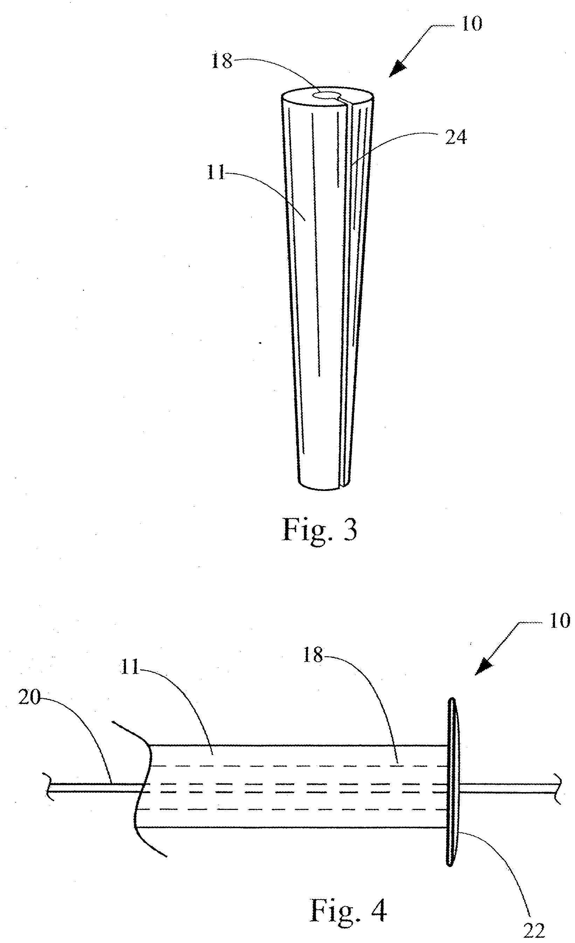

With reference now to Figure 3, still another embodiment of a medical device 10 of the present invention is shown. In this embodiment, the medical device 10 has a generally conical occluding member body 11, a lumen 18, and a longitudinal slit 24 extending throughout the length of the occluding member body 11. The longitudinal slit 24 extends from the external surface of the medical device 10 to the lumen 18, which is desirably in or near the center of the device 10. The longitudinal slit 24 is adapted to facilitate insertion of a wire guide into the lumen 18 laterally, rather than threading the wire guide through the lumen 18, as described above with reference to Figure 2.

With reference now to Figure 4, yet another embodiment of a medical device 10 of the present invention is shown. In this embodiment, the medical device 10 has a generally cylindrical occluding member body 11, a lumen 18 into which a wire guide 20 has been inserted, and a capping member 22. The capping member 22 may have any suitable shape and configuration to assist with anchoring the medical device 10 within a defect, as described in co-pending application serial no. 11/472,642 (Armstrong et al.), for example. In other embodiments, a capping member 22 may not be necessary to properly secure the medical device 10 within a defect, or a capping member 22 may be used in conjunction with another capping member on the opposite end of the medical device or with other suitable anchoring mechanisms to properly secure the medical device 10 within a defect.

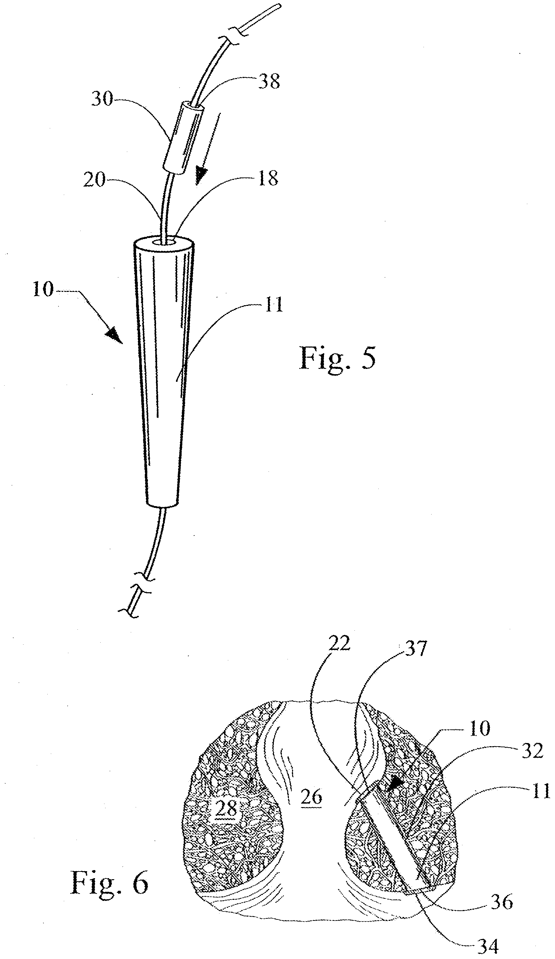

With reference now to Figure 5, a system for occluding a fistula is provided. In this embodiment, the system includes a wire guide 20, a medical device 10 comprising a generally conical occluding member body 11 having a first lumen 18, and a generally cylindrical pusher member 30 having a second lumen 38. Desirably, the occluding member body 11 and the pusher member 30 are configured to be received on the wire guide 20 (or other placement member) to facilitate insertion of the medical device 10 into a defect. The occluding member body 11 may be of any suitable shape, dimension, and material for at least partially occluding a defect in a patient. Similarly, the wire guide 20 and pusher member 30 may be of any suitable shape, dimension, and material for delivering the medical device 10 to its desired location. In the embodiment of Figure 5, the pusher member 30 comprises a generally tubular device with a central lumen therethrough.

With reference now to Figure 6, one embodiment of the medical device 10 of the present invention has been implanted into an anorectal fistula 32 located within the tissues surrounding the rectum 26 of the patient. The generally cylindrical occluding member body 11 of this embodiment extends from the primary opening 37 of the fistula 32 to the secondary opening 36 located in the perianal skin on the buttock 28 of the patient. In this embodiment, a capping member 22 and an anchoring member 34, such as a T-fastener, have been used to further secure the medical device 10 within the fistula. The capping member 22 is positioned adjacent to the primary opening 37, and the T-fastener 34 is positioned adjacent to the secondary opening 36. In other embodiments, only one anchoring member is used. In still other embodiments, no anchoring member is necessary to properly secure the medical device 10 within the fistula 32.

Turning now to a general discussion regarding methods for treating defects according to the present invention, suitable treatment methods include the steps of providing a medical device, such as any of those described herein, and implanting the medical device within a patient so that: (i) the medical device at least partially blocks the defect, for example at least the primary opening of a fistula, i.e., the primary opening and potentially one or more other segments of a fistula, such as the fistula tract and/or any secondary openings; (ii) the capping member(s) (if present) contacts portions of the tissues adjacent to the defect and/or portions of the tissues surrounding the openings of the defect; and (iii) the body of the medical device extends into at least a portion of the defect. The medical devices, systems, and methods of the present invention can be used to treat any defect in a mammalian body, such as a fistula having a primary opening in a wall of an alimentary canal. In some aspects, the invention provides medical devices and methods useful for blocking openings anywhere on or within the body of a patient, for example, blocking at least the primary opening of a urethro-vaginal fistulas, vesico-vaginal fistulas, tracheo-esophageal fistulas, gastro-cutaneous fistulas, fistulas occurring between the vascular and gastrointestinal systems, fistulas occurring between the vagina and the urethra or bladder, fistulas occurring between the anorectum and vagina, fistulas occurring between the vascular and gastrointestinal systems, defects in solid organs, defects in vessels, viscera containing persistent air leaks, any number of anorectal fistulas, such as recto-vaginal fistula, recto-vesical fistulas, recto-urethral fistulas, or recto-prostatic fistulas, or any other type of defect in a patient. Also, inventive devices and methods can be used to treat a defect regardless of its size and shape, and in some forms, are used to treat defects having a primary opening, secondary opening(s), and/or fistula tract with a diameter ranging from about 1 mm to about 20 mm, more typically from about 5 mm to about 10 mm.

Medical devices of the invention can be implanted using any suitable delivery method or placement technique. Illustratively, an occluding member body can be implanted by pushing or pulling the occluding member body into a suitable position within a fistula, either with or without the assistance of additional instrumentation, including but not limited to, catheters, wire guides, pusher members, probes, scopes, and the like. The implantation of the medical device may be facilitated by the use of visual, endoscopic, fluoroscopic, radiological, or CT guidance. The body of the occluding member may be secured at one or both ends by means of sutures, capping members, or any other suitable method of affixation, before, during, or after implantation of the medical device. The use of a capping member on each end of the medical device may be desirable to avoid the need for using sutures and piercing the tissues of the patient to firmly secure the medical device within the fistula tract. In some embodiments, at least one capping member is expandable so that it can be deployed in an un-expanded position and then expanded after the body of the medical device is properly positioned within the patient. In certain embodiments, a wire guide and catheter are used to cannulate the fistula before implantation.

As shown in Figure 5, one embodiment of the method of the present invention involves occluding a defect by using a placement member (such as a wire guide, endoscope, or hollow tubular member), an occluding member (such as a graft or plug) having a lumen therein, and a pusher member (such as those described herein) having a lumen therein. In some embodiments, the method of the present invention includes the steps of: inserting the placement member at least partially into the defect, advancing the placement member to a desired location within the patient, inserting the placement member into the lumen of the occluding member, and then advancing the occluding member to the desired location by inserting the placement member into the lumen of the pusher member and pushing the occluding member with the pusher member until the occluding member reaches the desired location within the patient. In some embodiments, a plug is pushed at least partially into a fistula with a pusher member, guided by a wire guide, by pushing a first end of the plug into the primary opening of the fistula and toward the secondary opening of the fistula, where the first end of the plug has a first diameter that is less than a second diameter of a second end of the plug. The plug may be pushed into the fistula until the second end of the plug becomes adequately secured within the primary opening of the fistula. The wire guide and pusher member may then be withdrawn from the fistula. In some embodiments, the placement member comprises a hollow tubular device, which may also be used to inject a contrast solution into the defect to facilitate visualization and implantation of the medical device.

In certain embodiments of the method of the present invention, a wire guide is inserted into the lumen of a plug by advancing it through a longitudinal slit in an external surface of the plug and into the lumen. In other embodiments, the wire guide or other placement member is inserted into the occluding member lumen by threading the wire guide or other placement member into the proximal end of the medical device and out through the distal end of the medical device, without the need for a slit in the surface of the device. In still other embodiments, the placement member is used to create a lumen through the body of the occluding member by applying sufficient force for the placement member to pierce the body of the occluding member, thereby creating a lumen.

In some embodiments of the present invention, the occluding member comprises a plug made from a dehydrated ECM material (such as the Anal Fistula Plug device from Cook Surgical, made of lyophilized porcine small bowel submucosa), and the plug is advanced to the desired location prior to rehydration. The plug may then be rehydrated by injecting a rehydrating fluid (such as saline) into the tissue surrounding the defect. Alternatively, the plug may be rehydrated prior to implantation or rehydrated *in situ* by absorption of fluid from surrounding tissues.

In the embodiment illustrated in Figure 6 (described more fully above), the method of the present invention involves occluding an anorectal fistula 32 within a patient. However, it will be understood that the devices, systems, and methods of the present invention may be useful in treating other types of defects as well. For example, the present invention may be used to occlude the lumens of vessels, such as arteries or veins, to prevent hemorrhage, or to divert blood flow.

More specifically, the methods of the present invention may be used to occlude the lumen of a hollow viscus, such as a leak in a bronchial stump after lobectomy or peumonectomy. An air leak may develop in a patient from a cut end of a bronchus after resectioning of a lung or lobe of a lung, resulting in a persistent pneumothorax. To treat such a defect, a wire guide may be inserted into the defect, desirably with the assistance of a bronchoscope. The bronchoscope may then be withdrawn, leaving the wire guide in place. An occluding member, such as a plug, may then be threaded onto the proximal end of the wire guide (or inserted laterally through a longitudinal slit in the occluding member) and advanced along the wire guide and toward the site of the air leak. Because the distal bronchus is disposed deep within the chest cavity of the patient, a pusher member is desirably used to push the placement member into the desired location within the bronchial defect to stop the air leak.

In the case of an entero-cutaneous fistula, the distal end of a wire guide may be inserted into the fistula tract and advanced toward the defect in the small bowel lumen, desirably using endoscopic or radiographic guidance. After the distal end of the wire guide is advanced to the desired location, an occluding member, such as a plug, may be slidably deployed from the proximal end of the wire guide, down the wire guide, and toward the defect. To advance the plug along the wire guide, a pusher member may be used. The use of a pusher member is especially desirable when the defect is in a relative inaccessible area of the body. The wire guide and pusher member may then be removed, leaving the plug implanted within the patient.

In another embodiment of the method of the present invention, a defect in a solid organ is treated. Following a biopsy of the liver (which entails inserting a needle through the skin of a patient and taking a small piece of the liver parenchyma), a hemorrhage may result. To occlude such a defect, a wire guide may be inserted through an access needle, and the distal end of the wire guide inserted into the depths of the bleeding biopsy site. An occluding member may then be threaded onto the proximal end of the wire guide and advanced down the wire guide and toward the bleeding biopsy site. Because the liver is within the peritoneal cavity at a relatively inaccessible site within the body, the occluding member is desirably pushed into position by using a pusher member until the occluding member reaches a desired position deep within the liver parenchyma to occlude the defect.

Methods of the present invention may include an endoscopic visualization (fistuloscopy) step, as disclosed in co-pending application Serial No. 10/945,634 (Armstrong), hereby incorporated by reference in its entirety. Such endoscopic visualization can be used, for example, to determine the shape and size of a defect, which in turn can be used to select an appropriately sized and shaped medical device for treating the defect. Illustratively, a thin flexible endoscope can be inserted into a secondary opening of a fistula and advanced under direct vision through the fistula tract and out through the primary opening. By performing fistuloscopy of a fistula, the primary opening can be accurately identified. Also, cleaning of the fistula can be performed prior to and/or during deployment of a medical device of the invention. For example, an irrigating fluid may be used to remove any inflammatory or necrotic tissue located within the fistula prior to implanting the medical device. In certain embodiments, one or more antibiotics are applied to the medical device and/or the soft tissues surrounding the fistula as an extra precaution or means of treating any residual infection within the fistula.

In some embodiments of the method of the present invention, after a defect is visualized using an endoscope, the endoscope is then used as a placement member. In desirable embodiments, the endoscope is long and thin. The proximal end of the endoscope may be placed inside the distal end of the occluding member body. The occluding member body may then be advanced along the endoscope until the endoscope protrudes from the proximal end of the occluding member body. A pusher member may then be threaded over the endoscope and used to facilitate placement of the medical device in the desired location in the patient.

The medical devices of the present invention can be modified before, during, and/or after deployment. Illustratively, the medical device may be cut, trimmed, sterilized, and/or treated (e.g., brought into contact, impregnated, coated, etc.) with one or more desirable compositions, such as any of those disclosed herein, e.g., anticoagulants (e.g., heparin), growth factors or other desirable property modifiers. In certain embodiments, following deployment of a medical device in accordance with the present invention, one or more portions of the medical device, for example, material protruding from the opening(s) of a defect, are trimmed off or otherwise removed. In other embodiments, where multiple or complex defects are present, multiple or composite medical devices may be implanted until all defects have been closed.

In certain embodiments, the medical device is anchored within the fistula by threading a securing device having a central lumen, over the tail of the medical device (if present) and securing it into position at skin level (e.g., by crimping it). In some embodiments, further anchoring of the medical device is achieved by using a material such as a small intestinal submucosa heterograft (a freeze-dried material that requires rehydration before use) for the medical device and inserting the medical device into the tract before the medical device material has been fully expanded by hydration. In other embodiments, autologous fibrin glue or other suitable adhesive is used in conjunction with the medical device to supplement the adhesive and occlusive properties of the disclosed invention (*e.g.,* Symphony PCS, DePuy AcroMed Inc.).

After the medical device is secured within a defect, such as an anorectal fistula, each end of the device may be trimmed to prevent any excess portions from protruding from the primary and/or secondary openings of the fistula after the procedure. As shown in Figure 6, the portions of the medical device 10 adjacent the secondary opening 36 and the primary opening 37 of the fistula have been trimmed to be flush with the secondary opening 36 and primary opening 37, respectively. In this embodiment, a capping member 22 and an anchoring member 34, such as a T-fastener, have been used to further secure the medical device 10 within the fistula. The capping member may be permanently attached to the occluding member body 11 or it may be configured to detach from the occluding member body 11 after a certain period of time sufficient for the occluding member body 11 to become ingrown into the fistula tract 32, as described in co-pending U.S. Application Serial No. 11/472,642 (Armstrong et al.). The capping member may be expandable or non-expandable and may be adjustable to various positions along the body of the occluding member.

All publications and patent applications cited in this specification are hereby incorporated by reference in their entirety, as if each individual publication or patent application were specifically and individually indicated to be incorporated by reference. Further, any theory, mechanism of operation, proof, or finding stated herein is meant to further enhance understanding of the present invention, and is not intended to limit the present invention in any way to such theory, mechanism of operation, proof, or finding. While the invention has been illustrated and described in detail in the drawings and foregoing description, the same is to be considered as illustrative and not restrictive in character, it being understood that only selected embodiments have been shown and described and that all equivalents, changes, and modifications that come within the spirit of the inventions as defined herein or by the following claims are desired to be protected.

## Claims

1. A medical device (10) for occluding a fistula, comprising:
a generally conical plug configured to be placed within a fistula and to occlude the fistula; and
a lumen (18) extending throughout the length of the plug;
wherein the lumen is configured to receive a wire guide (20)
**characterised by** a capping member (22) integral with, attached to or
otherwise associated with the plug and configured so as in use to prevent unintentional displacement of the plug.

2. The medical device of claim 1 wherein the lumen comprises a lumen positioned adjacent to a slit in an exterior surface of the plug, wherein the slit is configured to facilitate placement of the wire guide into the lumen.

3. The medical device of any of claims 1 to 2 wherein the plug comprises extracellular matrix material.

4. The medical device of any of claims 1 to 3 wherein the plug comprises dermal collagen.

5. The medical device of any of claims 1 to 4, wherein the plug comprises rolled extracellular matrix material.

6. The medical device of claim 1 or 5, further comprising a bioactive agent, wherein the bioactive agent is incorporated into or coated onto the medical device.

7. The medical device of claim 6, wherein the bioactive agent is a growth factor.

8. The medical device of claim 7, wherein the growth factor is selected from the group consisting of basic fibroblast growth factor (FGF-2), transforming growth factor beta (TGF -beta), epidermal growth factor (EGF), cartilage derived growth factor (CDGF) and platelet derived growth factor (PDGF).

9. The assembly of the medical device according to any one of the preceding claims with a wire guide and a pusher member, wherein the pusher member comprises a lumen throughout its length, wherein the wire guide is adapted for insertion into the lumen of the plug and into the lumen of the pusher member to allow advancement of the pusher member along the wire guide until the pusher member contacts a proximal end of the plug and pushes the plug with the pusher member.

10. The assembly of claim 9, wherein the pusher member is attached to a coupling member associated with the plug.

## Patentansprüche

1. Medizinische Vorrichtung (10) zum Okkludieren einer Fistel, umfassend:
einen allgemein konischen Plug, der dazu konfiguriert ist, in eine Fistel platziert zu werden und die Fistel zu okkludieren, und
ein Lumen (18), das sich durch die gesamte Länge des Plugs erstreckt,
wobei das Lumen zur Aufnahme einer Drahtführung (20) konfiguriert ist,
**gekennzeichnet durch** ein Abdeckglied (22), das mit dem Plug integral ist, daran angebracht oder ihm anderweitig zugeordnet ist sowie dazu konfiguriert ist, im Gebrauch eine unbeabsichtigte Verschiebung des Plugs zu verhindern.

2. Medizinische Vorrichtung nach Anspruch 1, wobei das Lumen ein Lumen umfasst, das neben einem Schlitz in einer äußeren Oberfläche des Plugs positioniert ist, wobei der Schlitz dazu konfiguriert ist, die Platzierung der Drahtführung in das Lumen zu erleichtern.

3. Medizinische Vorrichtung nach einem der Ansprüche 1 bis 2, wobei der Plug extrazelluläres Matrixmaterial umfasst.

4. Medizinische Vorrichtung nach einem der Ansprüche 1 bis 3, wobei der Plug dermales Kollagen umfasst.

5. Medizinische Vorrichtung nach einem der Ansprüche 1 bis 4, wobei der Plug aufgerolltes extrazelluläres Matrixmaterial umfasst.

6. Medizinische Vorrichtung nach Anspruch 1 oder 5, ferner umfassend ein bioaktives Mittel, wobei das bioaktive Mittel in die medizinische Vorrichtung eingegliedert oder darauf aufgetragen ist.

7. Medizinische Vorrichtung nach Anspruch 6, wobei das bioaktive Mittel ein Wachstumsfaktor ist.

8. Medizinische Vorrichtung nach Anspruch 7, wobei der Wachstumsfaktor aus der aus FGF-2 (basischem Fibroblasten-Wachstumsfaktor), TGF-beta (transformierendem Wachstumsfaktor beta), EGF (epidermalem Wachstumsfaktor), CDGF (Cartilage-derived growth factor) und PDGF (Platelet-derived growth factor) bestehenden Gruppe ausgewählt ist.

9. Anordnung der medizinischen Vorrichtung nach einem der vorhergehenden Ansprüche mit einer Drahtführung und einem Schieberglied, wobei das Schieberglied ein Lumen durch seine gesamte Länge hindurch umfasst, wobei die Drahtführung zum Einführen in das Lumen des Plugs und in das Lumen des Schieberglieds geeignet ist, um einen Vorschub des Schieberglieds entlang der Drahtführung zu gestatten, bis das Schieberglied ein proximales Ende des Plugs kontaktiert und den Plug mit dem Schieberglied schiebt.

10. Anordnung nach Anspruch 9, wobei das Schieberglied an einem dem Plug zugeordneten Koppelglied angebracht ist.

## Revendications

1. Dispositif médical (10) servant à l'occlusion d'une fistule, comprenant :
un bouchon globalement conique configuré pour être placé à l'intérieur d'une fistule et pour occlure la fistule ; et
une lumière (18) s'étendant sur toute la longueur du bouchon ;
la lumière étant configurée pour recevoir un fil-guide (20),
**caractérisé par** un élément formant coiffe (22) faisant partie intégrante du bouchon, étant fixé à celui-ci ou étant autrement associé à celui-ci, et configuré de façon à empêcher, pendant l'utilisation, un déplacement involontaire du bouchon.

2. Dispositif médical selon la revendication 1, dans lequel la lumière comprend une lumière positionnée de manière adjacente à une fente dans une surface extérieure du bouchon, dans lequel la fente est configurée pour faciliter le positionnement du fil-guide dans la lumière.

3. Dispositif médical selon l'une quelconque des revendications 1 et 2, dans lequel le bouchon comprend un matériau de matrice extracellulaire.

4. Dispositif médical selon l'une quelconque des revendications 1 à 3, dans lequel le bouchon comprend du collagène dermique.

5. Dispositif médical selon l'une quelconque des revendications 1 à 4, dans lequel le bouchon comprend un matériau de matrice extracellulaire enroulé.

6. Dispositif médical selon l'une quelconque des revendications 1 à 5, comprenant en outre un agent bioactif, dans lequel l'agent bioactif est incorporé dans le dispositif médical ou appliqué sur celui-ci de façon à former un revêtement.

7. Dispositif selon la revendication 6, dans lequel l'agent bioactif est un facteur de croissance.

8. Dispositif médical selon la revendication 7, dans lequel le facteur de croissance est sélectionné dans le groupe constitué du facteur de croissance basique des fibroblastes (FGF-2), du facteur croissance transformant bêta (TGF-bêta), du facteur de croissance épidermique (EGF), du facteur de croissance issu de cartilage (CDGF) et du facteur de croissance issu de plaquettes (PDGF).

9. Ensemble composé du dispositif médical selon l'une quelconque des revendications précédentes ainsi que d'un fil-guide et d'un organe poussoir, dans lequel l'organe poussoir comprend une lumière sur toute sa longueur, dans lequel le fil-guide est conçu pour être inséré dans la lumière du bouchon et dans la lumière de l'organe poussoir de façon à permettre l'acheminement de l'organe poussoir le long du fil-guide jusqu'à ce que l'organe poussoir vienne en contact avec une extrémité proximale du bouchon et pousse le bouchon au moyen de l'organe poussoir.

10. Ensemble selon la revendication 9, dans lequel l'organe poussoir est fixé à un organe d'accouplement associé au bouchon.
